# EUROPEAN PATENT SPECIFICATION

(11) **EP 3 560 909 B1**
(45) Date of publication and mention of the grant of the patent: **23.06.2021**
(21) Application number: 17886518.4
(22) Date of filing: 06.11.2017
(51) Int. Cl.: C07C 55/14, C07C 51/42, C07C 51/47

(54) **PRODUCTION METHOD FOR HIGH-QUALITY ADIPIC ACID**
HERSTELLUNGSVERFAHREN FÜR QUALITATIV HOCHWERTIGE ADIPINSÄURE
PROCÉDÉ DE PRODUCTION D'ACIDE ADIPIQUE DE HAUTE QUALITÉ

(30) Priority: 26.12.2016 CN 201611217560
(43) Date of publication of application: 30.10.2019
(73) Proprietor: Henan Shenma Nylon Chemical Co., Ltd., Pingdingshan, Henan 467013 (CN)
(72) Inventor: QIAO, Sihuai, Henan 467013 (CN); ZHAO, Duo, Henan 467013 (CN); CHEN, Juliang, Henan 467013 (CN); PAN, Qiang, Henan 467013 (CN); ZHONG, Ruxue, Henan 467013 (CN); LI, Yanli, Henan 467013 (CN); LIU, Zhenjiang, Henan 467013 (CN); GUO, Weidong, Henan 467013 (CN); SUN, Haojie, Henan 467013 (CN); OU, Ling, Henan 467013 (CN); JIN, Baoguo, Henan 467013 (CN); LIU, Shuixia, Henan 467013 (CN); GAO, Yang, Henan 467013 (CN); LV, Guohui, Henan 467013 (CN); REN, Jun, Henan 467013 (CN); LV, Zihao, Henan 467013 (CN); DONG, Feifei, Henan 467013 (CN)
(74) Representative: Lang, Christian
(86) International application number: PCT/CN2017/108868
(87) International publication number: WO 2018/121043

(56) References cited:
- CN-A- 104 130 119
- CN-A- 104 276 937
- CN-A- 106 810 442

## Description

The invention relates to the field of adipic acid production, and more particularly to a production method of high quality adipic acid including steps of tertiary purification separation, secondary activated carbon adsorption, tertiary filtration and so on.

Adipic acid is an aliphatic dibasic acid which has the common gender of an aliphatic dibasic acid, including chemical reaction, esterification reaction, amidation reaction and so on. Adipic acid is mainly used in the synthesis of industrial products such as nylon 66 salt, polyurethane, plasticizer and foaming agent. At present, the high-end products of adipic acid are gradually applied in civilian applications, such as food additives, civil silk, pharmaceutical intermediates and so on.

The traditional production process for adipic acid, its product specifications must comply with SH/T1499.1-2012 'pure adipic acid part 1: specification'. The purity and quality of adipic acid directly affect and restrict the application range of adipic acid. Therefore, improving the quality of adipic acid can expand the application field of adipic acid and further enhance market competitiveness.

CN1359364A discloses a method for preparing adipic acid crystal. The adipic acid crystals are dispersed in a liquid medium, the crystal shape is modified by stirring the liquid matrix to reduce crystal agglomeration and agglomeration properties and separate the liquid medium from the crystal. This method reduces the impurities entrained between the crystals, but the water-soluble trace impurities are still crystallized together with adipic acid. CN1867535A discloses a process for crystallizing adipic acid from an aqueous solution of adipic acid in nitric acid. The oxidation product is sequentially crystallized in several crystallizers having different crystallization temperatures, and solid crystals are harvested in the first crystallizer and the second crystallizer, respectively. This method considers that the crystallization of the excess three stages is not feasible, and proposes the advantage of being able to obtain refined adipic acid in the first two stages. The adipic acid industry now generally uses the method of secondary recrystallization to produce adipic acid. The process uses an excess of nitric acid to oxidize cyclohexanol, and the resulting cyclohexanol is dissolved in a 70-90°C nitric acid solution. After the primary recrystallization, the adipic acid crystallize out of the solution, and the crude adipic acid is obtained after separation, then subjected to second crystallization to obtain refined adipic acid. The requirements for product's color and trace impurities are getting higher and higher as users continue to improve the quality of the products. Nowadays, users are not satisfied with the conventional secondary-crystallized adipic acid products, but they are in urgent need of some high-end products. CN104130119A discloses a method for producing ultrapure adipic acid, which comprises the steps of: refining crude adipic acid for thickening, centrifuging crude adipic acid, crystallizing refined adipic acid for thickening, centrifuging refined adipic acid, and crystallizing ultrapure adipic acid for thickening and centrifuging ultrapure adipic acid to produce ultrapure adipic acid. The method theoretically reduces the nitrate impurities in the adipic acid product, but the mother liquor has a water balance problem, which makes it impossible to remove trace impurities, resulting in having no effect on improving product's melting color, UV value, purity and other indicators.

To overcome the defects of the prior art, one purpose of the invention is to provide a method for producing high quality adipic acid. On the basis of traditional double purification and separation, the invention uses three-stage purification and separation process to refine, two-stage activated carbon to adsorb soluble impurities, three-stage filtration to remove waste carbon impurities, water classification and water reuse. The method can greatly improve the quality index of adipic acid, and the obtained high quality adipic acid is widely used in high-end users such as civilian silk and TPU.

To achieve the above purpose, the invention adopts the following technical solutions.

Disclosed is a method for producing high quality adipic acid, which comprises a primary purification and separation process, a primary activated carbon decolorization process, a primary activated carbon filtration process, a secondary purification and separation process, a secondary activated carbon decolorization process, a secondary activated carbon filtration process, a ultrafine filter process and a tertiary purification and separation process; the specific process is as follows:
1) producing an adipic acid solution containing nitric acid through a nitric acid oxidation method, crystallizing adipic acid at a crystallization point of 65-75°C, and performing a primary purification and separation process at a temperature of 70-100°C, to yield crude adipic acid and primary hot water;
2) dissolving the crude adipic acid in water to obtain a solution A, adding an activated carbon slurry to the solution A for decolorization, filtering, crystallizing adipic acid at a crystallization point of 75-85°C, and performing a secondary purification and separation process at a temperature of 70-100°C, to yield refined adipic acid and secondary hot water;
3) dissolving the refined adipic acid in water to obtain a solution B, adding the activated carbon slurry to the solution B for secondary decolorization, filtering using with filter cloths of 200-300 mesh and 400-500 mesh, crystallizing adipic acid at a crystallization point of 75-85°C, and performing a tertiary purification and separation process at a temperature of 70-100°C, to yield high quality adipic acid and tertiary hot water.

Specifically, the tertiary hot water and the deionized water enter the primary water system; part of the water in the primary water system is used to dissolve the adipic acid in 3), and the other part enters the secondary water system together with the secondary hot water; part of the water in the secondary water system is used to dissolve the crude adipic acid in 2), and the other part enters the tertiary water system together with the primary hot water.

The mass concentration of the activated carbon slurry in 2) is 1-10%; the activated carbon slurry is measured by the dry weight of the activated carbon; 300-600g of activated carbon is added to per ton of solution A.

The mass concentration of the activated carbon slurry in 3) is 1-10%; the activated carbon slurry is measured by the dry weight of the activated carbon; 200-400g of activated carbon is added to per ton of solution A.

After the tertiary purification and separation process in 3), the impurities are removed through the ultrafine filter to prevent impurities from entering the next step, and finally the high quality adipic acid is obtained.

The adipic acid-containing solution produced by the nitric acid oxidation method is preferred, but is not limited to the production method in which a mixture of cyclohexanone and cyclohexanol, or cyclohexanol or cyclohexane is oxidized with nitric acid.

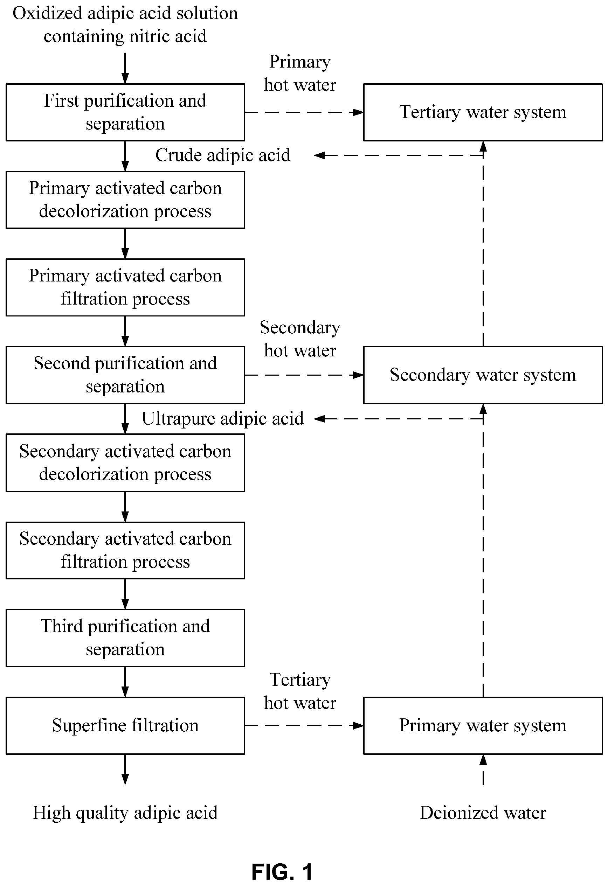

The ladder utilization characteristics of the water system are as follows: the tertiary hot water produced by the tertiary purification and separation process enters the primary water system; most of the water in the primary water system is used for the secondary dissolution of adipic acid (the water used to dissolve the refined adipic acid), and the remaining water enters the secondary water system together with the secondary hot water. The water in the secondary water system is also divided into two parts, most of the water is used for the primary dissolution of adipic acid (the water used to dissolve the crude adipic acid). The remaining water enters the primary water system together with the primary hot water (containing 20-30% nitric acid), which is then used as return water at the top of the concentrating tower for concentrating mother liquor. The primary hot water produced in the first purification and separation process contains nitric acid and by-products such as monobasic acid and dibasic acid, wherein the content of nitric acid is as high as 20-30%; the secondary hot water produced in the second purification and separation process contains 0.3-5% nitric acid; the tertiary hot water produced from the tertiary purification and separation process is substantially free of nitric acid. The three-stage water system realizes the ladder utilization of the mother liquor water, which effectively reduces the influence of nitric acid on the product quality during the preparation process. This system not only ensures the quality of adipic acid, but also realizes the rational use of water (see the dotted line in FIG. 1).

In the method of the disclosure, the tertiary hot water (substantially free of nitric acid) obtained during the tertiary purification and separation process is used as the water for dissolving adipic acid; the secondary hot water (about 0.3-5% nitric acid) produced from the secondary purification and separation process is used as the water for dissolving crude adipic acid; the primary hot water (containing 20-30% nitric acid) produced from the primary purification and separation process is used as reflux water at the top of the concentrating tower for concentrating mother liquor, which realizes the rational use of water during the preparation process. On the basis of tertiary purification and separation process, the method of the disclosure adds the adsorption decolorization and filtration process of activated carbon, so as to obtain adipic acid with higher purity and less impurities, and can be widely used not only for high-end products such as civilian silk and TPU. The invention successfully solves the quality problem of adipic acid existing in the traditional production process, reduces the impurities in the adipic acid, improves the quality of the product, expands the application field of the adipic acid, and increases the market competitiveness of the adipic acid.

The key point of the invention is to introduce the secondary adsorption and tertiary filtration processes of activated carbon. The crude adipic acid obtained from the primary purification and separation process is re-dissolved by adding hot water at 80-90°C. Then 1-10% of the activated carbon slurry with high efficiency is used for impurity adsorption, and the waste carbon after adsorbing impurities is subjected to primary filtration for realizing separation of the adipic acid solution and the waste activated carbon. After the adipic acid solution enters the second purification and separation process, the refined adipic acid obtained is re-dissolved by adding hot water at 80-90°C. Then 1-10% of the activated carbon slurry with high efficiency is used for impurity adsorption, and the waste carbon after adsorbing impurities is subjected to primary filtration for realizing separation of the adipic acid solution and the waste carbon after adsorbing impurities is subjected to secondary filtration for realizing separation of the adipic acid solution and the waste activated carbon. The ultrafine filter is used to remove the tertiary impurities, and the tertiary purification and separation process is used to obtain high-quality adipic acid, and finally the substantial improvement of the quality of the adipic acid is achieved.

Compared with the prior art, the beneficial effects of the invention are as follows:
1) The method has three processes, including tertiary purification and separation process, secondary activated carbon adsorption, and tertiary activated carbon filtration, so that the quality of adipic acid can be greatly improved;
2) The method satisfies the requirements for continuous production of high quality adipic acid in the industry;
3) The method improves the quality of the adipic acid, which changes the application of the adipic acid from industrial use to civilian use, and is used for producing polyester, TPU, carpet silk and civil silk, which improves the market competitiveness of the adipic acid.

FIG. 1 is a process flow chart of a method of production of high quality adipic acid according to one embodiment of the disclosure.

To further illustrate, embodiments detailing a method of production of high quality adipic acid are described below. It should be noted that the following embodiments are intended to describe and not to limit the disclosure.

### Example 1

The following is an example of using nitric acid to oxidize cyclohexanol to produce adipic acid. In the reactor, 6.0-6.5 mol nitric acid is reacted with 1 mol cyclohexanol to form 1 mol adipic acid, and the consumption of nitric acid is 2.6 mol. Due to the excessive addition of nitric acid, the formed adipic acid was dissolved in a nitric acid solution (30% nitric acid, 21% adipic acid, 8% impurities, and the rest is water). The production method of high quality adipic acid is how to extract adipic acid from the nitric acid solution, which is a process for removing impurities. The specific steps are as follows:
1) The nitric acid solution containing nitric acid, which is produced by the nitric acid oxidation method, is subjected to the primary purification and separation process at a controlled temperature of 85°C and obtain crude adipic acid (0.3-3% crude adipic acid, about 10% water, and 50 ppm impurity) having a crystallization point of 70°C and primary hot water;
2) the crude adipic acid is dissolved in water to obtain a solution A, which is then decolored by adding activated carbon slurry having a mass concentration of 5%, and the primary activated carbon is filtered off. The second purification and separation is carried out by controlling the temperature at 87°C and obtain refined adipic acid (25-40 ppm nitric acid, about 10% water) having a crystallization point of 80°C and secondary hot water; the activated carbon slurry is measured by the dry weight of the activated carbon; 500g of activated carbon is added to per ton of solution A.
3) the refined adipic acid is dissolved in water to obtain a solution B, which is then decolored by adding activated carbon slurry having a mass concentration of 5% (the activated carbon slurry is measured by the dry weight of the activated carbon; 300g activated carbon is added to per ton of solution A), and the secondary activated carbon is filtered off (filter with filter cloths of 100-200 mesh). The tertiary purification and separation process is carried out by controlling the temperature at 87°C to obtain high quality adipic acid (less than 5ppm nitric acid, about 10% water) having a crystallization point of 80°C and tertiary hot water; the refined adipic acid is further subjected to an ultrafine filter (equivalent to a filter cloths with 300-400 mesh, which can be directly purchased from a commercially available product) to remove trace impurities that is the insoluble solid. After drying, high quality adipic acid is obtained, wherein the nitrate is reduced by 70%, the ash is decreased by 25%, and the UV transmittance is increased by more than 3;
the tertiary hot water and the deionized water enter the tertiary water system; Part of the water in the primary water system is used to dissolve the adipic acid in 3), and the other part enters the secondary water system together with the secondary hot water; Part of the water in the secondary water system is used to dissolve the crude adipic acid in 2), and the other part enters the tertiary water system together with the primary hot water. The water in the tertiary water system acts as the condensed water at the top of the concentrating tower and eventually reaches the water balance.

### Example 2

The following is an example of the production of high quality adipic acid with an annual output of 100,000 tons/ton.

51 to 54% of a 86 t/h nitric acid solution was oxidized with 11.5 t/h of cyclohexanol in 10 reactors to obtain a solution of 21.3 wt. % adipic acid having a crystallization point of 71±1°C. After the first purification and separation process, crude adipic acid (10% water content) having a nitric acid content of 0.3 wt. % and 22 t/h is obtained. 25 t/h of hot water at 87°C from the secondary water system was added to prepare a solution of adipic acid at a concentration of 45 wt. %. After decolorization and filtration by the primary activated carbon, the secondary purification and separation is subjected to produce adipic acid having a nitric acid content of about 26 ppm and 21 t. Thereafter, 23.6 t/h of 87°C hot water from primary water system was added, and again, a concentration of 45 wt. % of adipic acid solution was prepared. After decolorization and filtration of the secondary activated carbon, tertiary purification and separation process is subjected to produce high quality adipic acid having a nitric acid content of 5 ppm or less. Some parameters of the production process not given are referred to in Example 1.

The following is a comparison table of the monthly average quality of adipic acid before and after using the method of the disclosure:

| Color | Color | Nitrate (ppm) | Dust (ppm) | UV transmittance |
|---|---|---|---|---|
| Before using the method of the disclosure | 0.924 | 2.1 | 2.45 | 91.5 |
| After using the method of the disclosure | 0.65 | 0.6 | 1.93 | 94.8 |

Mother liquid water system: 20 t/h of deionized water is added to the primary water system, mixed with the 16 t/h of tertiary water produced during the tertiary purification and separation process, and then passed into the primary water system. Among them, 23.6 t/h of water is used for dissolving refined adipic acid, the remaining 12.4 t/h of mother liquor water is added to the secondary water system, mixed with the 18 t/h of secondary hot water produced by the second purification and separation process. Among them, 25 t/h of water is used for dissolving crude adipic acid, and the remaining 5.4 t/h of mother liquor water enters the concentration process of mother liquor acid, which is used as return water at the top of the concentration tower to finally reach water balance.

## Claims

1. A method of producing adipic acid, comprising:
1) producing an adipic acid solution containing nitric acid through a nitric acid oxidation method, crystallizing adipic acid at a crystallization point of 65-75°C, and performing a primary purification and separation process at a temperature of 70-100°C, to yield crude adipic acid and primary hot water;
2) dissolving the crude adipic acid in water to obtain a solution A, adding an activated carbon slurry to the solution A for primary decolorization, filtering, crystallizing adipic acid at a crystallization point of 75-85°C, and performing a secondary purification and separation process at a temperature of 70-100°C, to yield refined adipic acid and secondary hot water;
3) dissolving the refined adipic acid in water to obtain a solution B, adding the activated carbon slurry to the solution B for secondary decolorization, filtering, crystallizing adipic acid at a crystallization point of 75-85°C, and performing a tertiary purification and separation process at a temperature of 70-100°C, to yield high quality adipic acid and tertiary hot water;
wherein
the tertiary hot water and deionized water enter a primary water system; part of the water in the primary water system is used to dissolve the adipic acid in 3), and the other part enters a secondary water system together with the secondary hot water; part of the water in the secondary water system is used to dissolve the crude adipic acid in 2), and the other part enters a tertiary water system together with the primary hot water.

2. The method of claim 1, **characterized in that** a mass concentration of the activated carbon slurry in 2) is 1-10%; the activated carbon slurry is measured by a dry weight of the activated carbon; 300-600 g of activated carbon is added to per ton of the solution A.

3. The method of claim 1, **characterized in that** a mass concentration of the activated carbon slurry in 3) is 1-10%; the activated carbon slurry is measured by a dry weight of the activated carbon; 200-400 g of activated carbon is added to per ton of the solution A.

4. The method of claim 1, **characterized in that** in 3), the filtering is performing by a fine filter.

## Patentansprüche

1. Verfahren zum Herstellen von Adipinsäure, umfassend:
1) Herstellen einer Adipinsäurelösung, die Salpetersäure durch ein Salpetersäure-Oxidationsverfahren enthält, Kristallisieren von Adipinsäure bei einem Kristallisationspunkt von 65-75 °C und Durchführen eines primären Reinigungs- und Trennprozesses bei einer Temperatur von 70-100 °C, um rohe Adipinsäure und primäres Warmwasser zu ergeben;
2) Lösen der rohen Adipinsäure in Wasser, um eine Lösung A zu erhalten, Hinzufügen einer Aktivkohle-Aufschlämmung zu der Lösung A zur primären Entfärbung, Filtern, Kristallisieren von Adipinsäure bei einem Kristallisationspunkt von 75-85 °C und Durchführen eines sekundären Reinigungs- und Trennprozesses bei einer Temperatur von 70-100 °C, um raffinierte Adipinsäure und sekundäres Warmwasser zu ergeben;
3) Lösen der raffinierten Adipinsäure in Wasser, um eine Lösung B zu erhalten, Hinzufügen der Aktivkohle-Aufschlämmung zu der Lösung B zur sekundären Entfärbung, Filtern, Kristallisieren von Adipinsäure bei einem Kristallisationspunkt von 75-85 °C und Durchführen eines tertiären Reinigungs- und Trennprozesses bei einer Temperatur von 70-100 °C, um hochwertige Adipinsäure und tertiäres Warmwasser zu ergeben;
wobei
das tertiäre Warmwasser und das entionisierte Wasser in ein Primäres-Wasser-System eintreten; ein Teil des Wassers im Primäres-Wasser-System verwendet wird, um die Adipinsäure in 3) aufzulösen, und der andere Teil zusammen mit dem sekundären Warmwasser in ein Sekundäres-Wasser-System ein; ein Teil des Wassers im Sekundäres-Wasser-System verwendet wird, um die rohe Adipinsäure in 2) aufzulösen, und der andere Teil zusammen mit dem primären Warmwasser in ein Tertiäres-Wasser-System eintritt.

2. Verfahren nach Anspruch 1, **dadurch gekennzeichnet, dass** eine Massenkonzentration der Aktivkohle-Aufschlämmung in 2) 1-10% beträgt; die Aktivkohle-Aufschlämmung durch ein Trockengewicht der Aktivkohle gemessen wird; 300-600 g Aktivkohle pro Tonne der Lösung A hinzugefügt wird.

3. Verfahren nach Anspruch 1, **dadurch gekennzeichnet, dass** eine Massenkonzentration der Aktivkohle-Aufschlämmung in 3) 1-10% beträgt; die Aktivkohle-Aufschlämmung durch ein Trockengewicht der Aktivkohle gemessen wird; 200-400 g Aktivkohle pro Tonne der Lösung A hinzugefügt wird.

4. Verfahren nach Anspruch 1, **dadurch gekennzeichnet, dass** in 3) das Filtern durch einen Feinfilter durchgeführt wird.

## Revendications

1. Procédé de production d'acide adipique, comprenant :
1) la production d'une solution d'acide adipique contenant de l'acide nitrique à l'aide d'un procédé d'oxydation d'acide nitrique, la cristallisation d'acide adipique à un point de cristallisation de 65-75°, et la réalisation d'une opération de purification et de séparation primaire à une température de 70-100°C, pour l'obtention d'acide adipique brut et d'eau chaude primaire ;
2) la dissolution de l'acide adipique brut dans de l'eau pour l'obtention d'une solution A, l'ajout d'une boue de charbon actif à la solution A pour une décoloration primaire, le filtrage, la cristallisation d'acide adipique à un point de cristallisation de 75-85°C, et la réalisation d'une opération de purification et de séparation secondaire à une température de 70-100°C, pour l'obtention d'acide adipique raffiné et d'eau chaude secondaire ;
3) la dissolution de l'acide adipique raffiné dans de l'eau pour l'obtention d'une solution B, l'ajout de la boue de charbon actif à la solution B pour une décoloration secondaire, le filtrage, la cristallisation d'acide adipique à un point de cristallisation de 75-85°C, et la réalisation d'une opération de purification et de séparation tertiaire à une température de 70-100°C, pour l'obtention d'acide adipique de grande qualité et d'eau chaude tertiaire ;
dans lequel
l'eau chaude tertiaire et de l'eau désionisée entrent dans un système d'eau primaire ; une partie de l'eau dans le système d'eau primaire est utilisée pour dissoudre l'acide adipique dans l'étape 3), et l'autre partie entre dans un système d'eau secondaire ensemble avec l'eau chaude secondaire ; une partie de l'eau dans le système d'eau secondaire est utilisée pour dissoudre l'acide adipique brut dans l'étape 2), et l'autre partie entre dans un système d'eau tertiaire ensemble avec l'eau chaude primaire.

2. Procédé selon la revendication 1, **caractérisé en ce qu'**une concentration massique de la boue de charbon actif dans l'étape 2) est de 1-10% ; la boue de charbon actif est mesurée par un poids sec du charbon actif ; 300-600 g de charbon actif sont ajoutés par tonne de solution A.

3. Procédé selon la revendication 1, **caractérisé en ce qu'**une concentration massique de la boue de charbon actif dans l'étape 3) est de 1-10% ; la boue de charbon actif est mesurée par un poids sec du charbon actif ; 200-400 g de charbon actif sont ajoutés par tonne de solution A.

4. Procédé selon la revendication 1, **caractérisé en ce que** dans l'étape 3), le filtrage est réalisé à l'aide d'un filtre fin.
